# EUROPEAN PATENT SPECIFICATION

(11) **EP 3 866 740 B1**
(45) Date of publication and mention of the grant of the patent: **20.04.2022**
(21) Application number: 19762930.6
(22) Date of filing: 30.08.2019
(51) Int. Cl.: A61F 13/42, G01N 27/403, B60C 19/00

(54) **CONSTRUCTIONAL FEATURES OF A MOISTURE DETECTOR, AND METHOD FOR MAKING SUCH**
KONSTRUKTIONSMERKMALE EINES FEUCHTIGKEITSDETEKTORS UND VERFAHREN ZU SEINER HERSTELLUNG
CARACTÉRISTIQUES DE CONSTRUCTION D'UN DÉTECTEUR D'HUMIDITÉ ET SON PROCÉDÉ DE FABRICATION

(30) Priority: 15.10.2018 NO 20181323
(43) Date of publication of application: 25.08.2021
(73) Proprietor: Fyster, As, 1266 Oslo (NO)
(72) Inventor: ECKHOFF, Rolf, 0260 Oslo (NO)
(74) Representative: Onsagers AS
(86) International application number: PCT/EP2019/073240
(87) International publication number: WO 2020/078607

(56) References cited:
- EP-A1- 2 336 757
- EP-A1- 3 007 913
- JP-A- 2006 053 057
- US-A- 4 492 622

## Description

### Introduction

The invention relates to constructional features of a moisture detector comprising a dry charged catalytic accumulator as the detecting element as well as a method for making such. The constructional features provide optimized power generation when the dry charged catalytic accumulator is exposed to moisture.

### Background

The applicant has previously developed a moisture detector that can be inactive for several years without any weakening in its functionality. The solution is based on using a dry charged accumulator as both the means for detecting moisture and the means for generating power necessary for indicating detection of moisture. It is thus a totally independent solution that is self-powered and self-driven. When power is generated due to activation of the dry charged accumulator by the ingress of water, connected electronics such as a transmitter can be activated for indicating detection of moisture, e.g. by transmitting a warning signal.

EP 3007913 B1, hereby included as reference, describes said solution where a moisture detector comprising a dry charged catalytic accumulator is used for detecting wear of a tire. When the tire is worn to a certain degree, water will at some point penetrate and activate the dry charged accumulator. This will then start generating power enabling a transmitter to transmit a signal indicating detection of moisture.

US 2013336842 A1 describes a moisture detector for controlling humidity. The moisture detector is designed for having high water absorption rates and thus high sensitivity and short response time. The sensor comprises a dielectric material adapted for absorbing surrounding humidity. The construction described is unsuited for use in an embodiment where a dry charged catalytic accumulator is used for detecting moisture.

When a dry charged accumulator is exposed to moisture, the amount of moisture it is exposed to may determine the amount of power generated.

If the dry charged accumulator is submerged in water, it will immediately start producing maximum power determined by the material of the electrodes and the electrolyte, e.g. a copper electrode and a zinc electrode with dry salt between them as an electrolyte.

If, on the other hand, the amount of moisture the dry charged accumulator is exposed to is small, such when for instance only a part of the dry charged accumulator is made wet, the dry charged accumulator may not produce enough power for sufficiently powering connected electronics, e.g. a transmitter.

JP 2006053057 A describes a water leak sensor and system where a threshold value for detecting water leaks can be adjusted by applying different water absorber amounts in an absorber. The absorber is an outer layer of a housing of the water leak sensor that will exposed to water when the absorber is saturated. The outer layer is in direct contact with an opening between electrodes. When water penetrates this layer, energy is generated. This construction is exposed to leakage currents when the absorber is partly saturated.

US 4492622 A describes an electro-chemical cell suited for measuring moisture. The construction described is also exposed to leakage currents.

EP 2336757 A1 describes a moisture sensor comprising electrodes and a compound arranged between the electrodes. When the compound is exposed to water, impedance change is measured.

Prior art solutions describing sensors that are activated when exposed to moisture and water are susceptible to leakage currents, which make it difficult to control the activation of the sensors.

Problems with prior art are solved by the present invention where the dry charged accumulator is encapsulated in a material having absorbing properties such that moisture is accumulated in the material over time and released when sufficiently saturated. An intermediate layer having absorption properties is placed between electrodes of the dry charged accumulator. The intermediate layer will dissolve when the material having absorbing properties is sufficiently saturated, thereby exposing the dry charged accumulator to sufficient amount of humidity for sufficiently powering connected electronics such as for instance a transmitter.

This solution will avoid leakage current from the dry charged accumulator when not exposed to a minimum level of humidity.

### Summary of the invention

The invention is set forth in the independent claims and the dependent claims describe alternatives of the invention.

The object of the present invention is to provide an improved moisture detector according to independent claim 1 comprising a dry charged catalytic accumulator as the detecting element as well as a method for making such a moisture detector according to independent claim 7. The constructional features provide optimized power generation when the dry charged catalytic accumulator is exposed to moisture.

The moisture detector according to the invention comprises a dry charged catalytic electrode accumulator as means for generating energy when moisture is detected. The moisture detector comprises an absorbing material encapsulating at least a part of the dry charged catalytic electrode accumulator, where the absorbing material has spongy absorption properties.

An intermediate layer having absorption properties is placed between electrodes of the dry charged catalytic electrode accumulator.

According to the invention, the moisture detector comprises a thin membrane between the absorbing material and the dry charged catalytic electrode accumulator, where the thin membrane is made of a material dissolving when exposed to a humidity of at least 75% RH.

The thin membrane will ensure that the dry charged catalytic electrode accumulator will be activated only when the thin membrane dissolve. This will happen when the absorbing material having the spongy absorption properties is sufficiently saturated with humidity, having a relative humidity of at least 75%. This avoids leakage currents in the dry charged catalytic electrode accumulator when exposed to only small amounts of humidity.

In one embodiment, the absorbing material is made of a formulation of Poly Vinyl Alcohol (PVOH), while in a differing formulation the thin membrane is also made of PVOH, treated with polymer plasticisers with varying degrees of hygroscopicity. The invention is further defined by a method for making a moisture detector by providing a dry charged catalytic electrode accumulator as means for generating energy when moisture is detected. The method is characterized in encapsulating at least a part of the dry charged catalytic electrode accumulator in an absorbing material having spongy absorption properties, and adding an intermediate layer, having absorption properties, between electrodes of the dry charged catalytic electrode accumulator.

According to the invention, the method is characterised in adding a thin membrane between the absorbing material and the dry charged catalytic electrode accumulator, where the thin layer is made of a material dissolving when exposed to a humidity of at least 75% RH.

Further features of the invention are defined in the dependent claims.

### Detailed description of the invention

In the following, embodiments of the invention will be discussed in more detail with reference to the appended drawings showing examples of different embodiment. It should be understood, however, that the drawings are not intended to limit the invention to the subject-matter depicted in the drawings.

Figure 1 is a cross-sectional view of a moisture detector encapsulated by an absorbing material, and

Figure 2 is an isometric view of the moisture detector.

The applicant has previously developed a simple and inexpensive self-powered solution for detecting and indicating if moisture has penetrated a tire. The purpose is to indicate wear of a tire. The solution generates its own power when humidity penetrates a tire, thereby reaching a catalytic electrode accumulator which is installed below the wear surface in the tread area of a tire. Ingress of moisture will happen when the tire has been worn to a certain degree and is exposed to the moisture. This will then activate the accumulator. The power generated can be used for powering a transmitter used for warning that the tire has been worn. The moisture detector can be inactive for several years without any degrading of its functionality.

The present invention may be used as an addition to the said moisture detector for improving its detection properties, as well as an improvement to other moisture detectors based on a dry charged catalytic accumulator for detecting and indicating moisture.

The embodiments described and shown in the figures are based on one of applicant's previous moisture detectors which is based on a dry charged catalytic accumulator. These are however only examples, and not embodiments which the invention is restricted to.

The present invention is defined by a moisture detector comprising a dry charged catalytic accumulator as the means for generating energy when moisture is detected. The moisture detector comprises an absorbing material encapsulating at least a part of the dry charged catalytic electrode accumulator, where the absorbing material has spongy absorption properties.

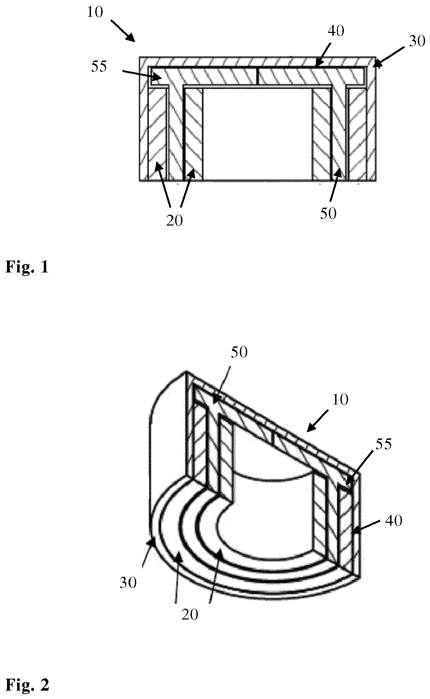

Figure 1 shows an embodiment of a moisture detector 10 according to the invention, where an absorbing material 30 is encapsulating all parts of the dry charged catalytic electrode accumulator 20. The absorbing material 30 is made in a material having spongy absorption properties making it sponge up humidity until it is fully saturated. When saturated, it will release the humidity which then will activate the dry charged catalytic electrode accumulator 20.

The moisture detector shown in figure 1 is an example of an embodiment used for detecting wear of a tire. This construction comprises a tube-shaped dry charged catalytic electrode accumulator 20 made from different layers. One layer comprises a cathode material, and another layer comprises an anode material. Between these layers there is an intermediate layer 50 made in a material having absorption properties. Electronics for warning detection of moisture, i.e. due to activation of the dry charged catalytic electrode accumulator 20 generating energy, is connected (not shown) between the anode and cathode of the dry charged catalytic electrode accumulator 20.

The intermediate layer 50 may for instance be a textile saturated with salt crystals, e.g. NaCl. In the embodiment shown in figure 1, parts of the intermediate layer 50 protrude from the layered structure. The protruding part or wing part 55 of the intermediate layer 50 may be divided into several wings which may be the first contact point with moisture.

The intermediate layer 50 acts as a trigger for activating the dry charged catalytic electrode accumulator 20 for generating energy, ref. EP 3007913 B1.

If the intermediate layer 50 is only made partly wet, it may not sufficiently trigger the dry charged catalytic electrode accumulator 20. Only leak currents may be produced which is insufficient for powering and activating connected electronics. This is avoided by the present invention where the moisture detector, in addition to the dry charged catalytic electrode accumulator 20, comprises an absorbing material 30 encapsulating the dry charged catalytic electrode accumulator 20, where the absorbing material 30 has spongy absorption properties.

In one embodiment, the absorbing material 30 is made of a formulation of Poly Vinyl Alcohol (PVOH).

Figure 2 shows an isometric view of the construction of the moisture detector shown in figure 1.

In one embodiment of the invention, there is also a protection layer around the absorbing material 30, made of PVOH and a variety of polymer additives such as (but not limited to) silicone, fatty acid amides or chlorides, which give hydrophobic qualities to the surface of PVOH.

In an embodiment there is provided a thin membrane 40 between the absorbing material 30 and the dry charged catalytic electrode accumulator 20, where the thin membrane 40 is made of a material dissolving when exposed to sufficient amounts of humidity, at least 75% RH. This will ensure that there is a delay before humidity is absorbed by the intermediate layer 50.

The invention is also defined by a method for providing a moisture detector 10. The method comprises: providing a dry charged catalytic electrode accumulator 20 as means for generating energy when moisture is detected. This may for instance be the one described in the example above. The next step is encapsulating at least a part of the dry charged catalytic electrode accumulator 20 in an absorbing material 30 having spongy absorption properties.

In one embodiment, the method comprises using Poly Vinyl Alcohol (PVOH) and polymer additives as components in the absorbing material. The polymer additives used may in one embodiment be one or more of the following materials: silicon, fatty acid amides and chlorides as the polymer additives.

According to the invention, an intermediate step is performed. This is adding a thin membrane 40 between the absorbing material 30 and the dry charged catalytic electrode accumulator 20, where the thin membrane 40 is made of a material dissolving when exposed to sufficient amounts of humidity, at least 75% RH. The material used for the thin membrane 40 is in one embodiment Poly Vinyl Alcohol (PVOH) and polymer additives as components in the thin membrane. Examples of polymer additives used is one or more of the following materials: silicon, fatty acid amides and chlorides.

An additional step is treating the thin membrane 40 with polymer plasticisers with varying degrees of hygroscopicity.

In the preceding description, various aspects of a moisture detector comprising a dry charged catalytic electrode accumulator according to the invention have been described with reference to the illustrative embodiment. For purposes of explanation, the exemplified embodiments were set forth in order to provide a thorough understanding of the moisture sensor and a method for making such.

As understood from the description above, the constructional features defining the invention can be applied to moisture detectors used for other purposes than detecting wear in tires.

Examples are moisture detectors used for humidity detection in buildings and materials like in bathrooms and basements, in boats, aeroplanes, oil and gas drilling platforms, hollow section structural members in bridges and other technical installations etc, where the ingress of a significant level of moisture might have a detrimental effect on the fabric and/or the structure of the host installation.

## Claims

1. A moisture detector (10) comprising:
- a dry charged catalytic electrode accumulator (20) as means for generating energy when moisture is detected;
- an absorbing material (30) encapsulating at least a part of the dry charged catalytic electrode accumulator (20), where the absorbing material (30) has spongy absorption properties;
- an intermediate layer (50) having absorption properties placed between electrodes of the dry charged catalytic electrode accumulator (20),
**characterized in that** there is a thin membrane (40) between the absorbing material (30) and the dry charged catalytic electrode accumulator (20), where the thin membrane (40) is made of a material dissolving when exposed to humidity of at least 75% RH.

2. The moisture detector (10) according to any one of the claims 1, **characterized in that** the absorbing material (30) is made of Poly Vinyl Alcohol (PVOH) and polymer additives.

3. The moisture detector (10) according to claim 2,
**characterized in that** the polymer additives are one or more of the following materials: silicon, fatty acid amides or chlorides.

4. The moisture detector (10) according to one of the claims 1 to 3, **characterized in that** the thin membrane (40) is made of Poly Vinyl Alcohol (PVOH) and polymer additives.

5. The moisture detector (10) according to claim 4,
**characterized in that** the polymer additives are one or more of the following materials: silicon, fatty acid amides or chlorides.

6. The moisture detector (10) according to1 to 5,
**characterized in that** the thin membrane (40) is treated with polymer plasticisers with varying degrees of hygroscopicity.

7. A method for making a moisture detector (10) comprising:
providing a dry charged catalytic electrode accumulator (20) as means for generating energy when moisture is detected,
- encapsulating at least a part of the dry charged catalytic electrode accumulator (20) in an absorbing material (30) having spongy absorption properties;
- adding an intermediate layer (50), having absorption properties, between electrodes of the dry charged catalytic electrode accumulator (20),
**characterized in** adding a thin membrane (40) between the absorbing material (30) and the dry charged catalytic electrode accumulator (20), where the thin membrane (40) is made of a material dissolving when exposed to a humidity of at least 75% RH.

8. The method according to claim 7,
**characterized in** using Poly Vinyl Alcohol (PVOH) and polymer additives as components in the absorbing material (30).

9. The method according to claim 8, **characterized in** using one or more of the following materials: silicon, fatty acid amides or chlorides as the polymer additives.

10. The method according to one of the claims 7 to 9,
**characterized in** using Poly Vinyl Alcohol (PVOH) and polymer additives as components in the thin membrane (40).

11. The method according to claim 10, **characterized in** using one or more of the following materials: silicon, fatty acid amides or chlorides as polymer additives.

12. The method according to claim 10 or 11, **characterized in** treating the thin membrane (40) with polymer plasticisers with varying degrees of hygroscopicity.

## Patentansprüche

1. Feuchtigkeitsdetektor (10) umfassend:
- einen trockenen, geladenen, katalytischen Elektrodenakkumulator (20) als Mittel zur Erzeugung von Energie, wenn Feuchtigkeit festgestellt wird;
- ein absorbierendes Material (30), das mindestens einen Teil des trockenen, geladenen, katalytischen Elektrodenakkumulators (20) einkapselt, wobei das absorbierende Material (30) schwammartige Absorptionseigenschaften aufweist;
- eine Zwischenschicht (50) mit Absorptionseigenschaften, die zwischen den Elektroden des trockenen, geladenen, katalytischen Elektrodenakkumulators (20) angeordnet ist,
**dadurch gekennzeichnet, dass** eine dünne Membran (40) zwischen dem absorbierenden Material (30) und dem trockenen, geladenen, katalytischen Elektrodenakkumulator (20) vorhanden ist, wobei die dünne Membran (40) aus einem Material besteht, das sich auflöst, wenn es einer Feuchtigkeit von mindestens 75 % RH ausgesetzt wird.

2. Feuchtigkeitsdetektor (10) nach einem der Ansprüche 1, **dadurch gekennzeichnet, dass** das absorbierende Material (30) aus Polyvinylalkohol (PVOH) und Polymeradditiven hergestellt ist.

3. Feuchtigkeitssensor (10) nach Anspruch 2, **dadurch gekennzeichnet, dass** die Polymeradditive eines oder mehrere der folgenden Materialien sind: Silizium, Fettsäureamide und - chloride.

4. Feuchtigkeitssensor (10) nach einem der Ansprüche 1 bis 3, **dadurch gekennzeichnet, dass** die dünne Membran (40) aus Polyvinylalkohol (PVOH) und Polymeradditiven hergestellt ist.

5. Feuchtigkeitsdetektor (10) nach Anspruch 4, **dadurch gekennzeichnet, dass** die Polymeradditive eines oder mehrere der folgenden Materialien sind: Silizium, Fettsäureamide oder - chloride.

6. Feuchtigkeitsdetektor (10) nach einem der Ansprüche 1 bis 5, **dadurch gekennzeichnet, dass** die dünne Membran (40) mit polymeren Weichmachern mit unterschiedlichem Grad an Hygroskopizität behandelt ist.

7. Verfahren zur Herstellung eines Feuchtigkeitsdetektors (10), umfassend:
Bereitstellen eines trockenen, geladenen, katalytischen Elektrodenspeichers (20) als Mittel zur Energieerzeugung, wenn Feuchtigkeit festgestellt wird,
Einkapseln mindestens eines Teils des trockenen, geladenen, katalytischen Elektrodenackumulators (20) in ein absorbierendes Material (30) mit schwammartigen Absorptionseigenschaften;
Hinzufügen einer Zwischenschicht (50) mit Absorptionseigenschaften zwischen Elektroden des trockenen, geladenen, katalytischen Elektrodenakkumulators (20),
**dadurch gekennzeichnet, dass** eine dünne Membran (40) zwischen dem absorbierenden Material (30) und dem trockenen, geladenen, katalytischen Elektrodenakkumulator (20) hinzugefügt wird, wobei die dünne Membran (40) aus einem Material hergestellt ist, das sich auflöst, wenn es einer Feuchtigkeit von mindestens 75 % RH ausgesetzt wird.

8. Verfahren nach Anspruch 7, **dadurch gekennzeichnet, dass** Polyvinylalkohol (PVOH) und Polymeradditive als Komponenten in dem absorbierenden Material (30) verwendet werden.

9. Verfahren nach Anspruch 8, **dadurch gekennzeichnet, dass** eines oder mehrere der folgenden Materialien verwendet werden: Silizium, Fettsäureamide oder -chloride als Polymeradditive.

10. Verfahren nach einem der Ansprüche 7 bis 9, **dadurch gekennzeichnet, dass** Polyvinylalkohol (PVOH) und Polymeradditive als Komponenten in der dünnen Membran (40) verwendet werden.

11. Verfahren nach Anspruch 10, **dadurch gekennzeichnet, dass** eines oder mehrere der folgenden Materialien verwendet werden: Silizium, Fettsäureamide oder -chloride als Polymeradditive.

12. Verfahren nach Anspruch 10 oder 11, **dadurch gekennzeichnet, dass** die dünne Membran (40) mit polymeren Weichmachern mit unterschiedlichem Grad an Hygroskopizität behandelt wird.

## Revendications

1. Détecteur d'humidité (10) comprenant :
- un accumulateur catalytique à électrodes chargé à sec (20) en tant que moyen de génération d'énergie lorsque de l'humidité est détectée ;
- un matériau absorbant (30) encapsulant au moins une partie de l'accumulateur catalytique à électrodes chargé à sec (20), où le matériau absorbant (30) a des propriétés d'absorption spongieuse ;
- une couche intermédiaire (50) ayant des propriétés d'absorption placée entre les électrodes de l'accumulateur catalytique à électrodes chargé à sec (20),
**caractérisé en ce qu'**il y a une membrane fine (40) entre le matériau d'absorption (30) et l'accumulateur catalytique à électrodes chargé à sec (20), où la membrane fine (40) est formée par un matériau qui se dissout lorsqu'il est exposé à une humidité d'au moins 75 % HR.

2. Détecteur d'humidité (10) selon l'une quelconque des revendications 1,
**caractérisé en ce que** le matériau absorbant (30) est formé d'alcool polyvinylique (PVOH) et d'additifs polymères.

3. Détecteur d'humidité (10) selon la revendication 2,
**caractérisé en ce que** les additifs polymères sont l'un ou plusieurs des matériaux suivants : silicium, amides d'acides gras ou chlorures.

4. Détecteur d'humidité (10) selon l'une des revendications 1 à 3,
**caractérisé en ce que** la membrane fine (40) est formée d'alcool polyvinylique (PVOH) et d'additifs polymères.

5. Détecteur d'humidité (10) selon la revendication 4,
**caractérisé en ce que** les additifs polymères sont l'un ou plusieurs des matériaux suivants : silicium, amides d'acides gras ou chlorures.

6. Détecteur d'humidité (10) selon 1 à 5,
**caractérisé en ce que** la membrane fine (40) est traitée avec des plastifiants polymères avec des degrés d'hygroscopie variables.

7. Procédé de fabrication d'un détecteur d'humidité (10) comprenant :
- la fourniture d'un accumulateur catalytique à électrodes chargé à sec (20) en tant que moyen de génération d'énergie lorsque de l'humidité est détectée,
- l'encapsulation d'au moins une partie de l'accumulateur catalytique à électrodes chargé à sec (20) dans un matériau absorbant (30) ayant des propriétés d'absorption spongieuse ;
- l'ajout d'une couche intermédiaire (50), ayant des propriétés d'absorption, entre les électrodes de l'accumulateur catalytique à électrodes chargé à sec (20),
**caractérisé par** l'ajout d'une membrane fine (40) entre le matériau d'absorption (30) et l'accumulateur catalytique à électrodes chargé à sec (20), où la membrane fine (40) est formée par un matériau qui se dissout lorsqu'il est exposé à une humidité d'au moins 75 % HR.

8. Procédé selon la revendication 7,
**caractérisé par** l'utilisation d'alcool polyvinylique (PVOH) et d'additifs polymères en tant que composants dans le matériau absorbant (30).

9. Procédé selon la revendication 8, **caractérisé par** l'utilisation d'un ou plusieurs des matériaux suivants : silicium, amides d'acides gras ou chlorures en tant qu'additifs polymères.

10. Procédé selon l'une des revendications 7 à 9,
**caractérisé par** l'utilisation d'alcool polyvinylique (PVOH) et d'additifs polymères en tant que composant dans la membrane fine (40).

11. Procédé selon la revendication 10, **caractérisé par** l'utilisation d'un ou plusieurs des matériaux suivants : silicium, amides d'acides gras ou chlorures en tant qu'additifs polymères.

12. Procédé selon la revendication 10 ou 11, **caractérisé par** le traitement de la membrane fine (40) avec des plastifiants polymères avec des degrés d'hygroscopie variables.
